# EUROPEAN PATENT APPLICATION

(11) **EP 2 574 929 A1**
(43) Date of publication of application: **03.04.2013**
(21) Application number: 11183032.9
(22) Date of filing: 28.09.2011
(51) Int. Cl.: G01N 33/574

(54) **Marker in diagnosing prostate cancer (PC)**

(71) Applicant: IMG Institut für medizinische Genomforschung Planungsgesellschaft M.B.H., 1010 Vienna (AT)
(72) Inventor: Klocker, Helmut, 6401 Inzing (AT); Schäfer, Georg, 6020 Innsbruck (AT); Chui-Pressinotti, Nicole, 65185 Wiesbaden (DE); Sültmann, Holger, 67117 Limburgerhof (DE); Kuner, Ruprecht, 73760 Ostfildern (DE); Fälth, Maria, 26393 Höganäs (SE)
(74) Representative: Sonn & Partner Patentanwälte

(57) **Abstract**

The invention discloses the use of Maternal Embryonic Leucine Zipper Kinase (MELK) as marker for diagnosis of prostate cancer (PC) in a tissue sample or in a body fluid sample, especially for the diagnosis of high-risk PC.

## Description

The present invention relates to a marker in diagnosing prostate cancer (PC).

Patient care in PC is still a challenge with respect to the assessment of clinically relevant disease course and specific therapies. Present parameters in the routine diagnostics like Gleason score or PSA serum levels have a limited accuracy to indicate a severe cancer disease and worse outcome.

Measuring prostate specific antigen (PSA) has been a matter of routine to detect prostate cancer, but is insufficient to distinguish between different tumour grades. The widespread use of PSA for the detection of PC has resulted in an increasing number of men diagnosed with organ-confined, low Gleason-score PC, which are potentially curable.

However, the high sensitivity of the PSA test is accompanied by a low specificity, causing many patients suffering from unnecessary biopsy taking. Men with normal prostate pathology generally have a PSA level in blood below 4ng/ml. PSA levels between 4ng/ml and 10ng/ml ("grey zone") have a 25% chance of having prostate cancer. However, some men with PC may have normal levels of PSA and elevated levels of PSA may also be due to other factors, such as age, infection etc. As a consequence, in 75% of the cases, men with an abnormal DRE and a PSA in this grey zone have a negative, or a seemingly unnecessary biopsy.

The Gleason Grading System is commonly used for histological-based grading of PC tissue. Since prostate tumours are often multifocal, the Gleason Score (GS) is the sum of the two most prevalent tumour patterns, which are graded 1 (CA1) as the most differentiated to 5 (CA5) as the least differentiated pattern of cancerous gland.

The discrimination between different tumour grades is important with respect to treatment decisions: Currently, many men who are diagnosed with GS 6 PC are often "over"-treated and risk suffering from urinary and sexual dysfunction - frequent effects of treatment. Therefore, it is also important to develop a sensitive and specific diagnostic tool to distinguish between different tumour grades. To address this problem, many groups have recently started to profile expression levels of PC to identify deregulated genes during disease progression. However, although many of these have addressed the question of molecular differences between normal, tumour, benign prostatic hypertrophy (BPH), and the putative precursor lesion prostatic intraepithelial neoplasia (PIN), little is still known about molecular changes between low- and high-risk tumours. One hallmark of highly aggressive tumours is their resistance towards apoptosis. Defects in the apoptotic signalling machinery are a key factor for the survival of malignant cells, and also contribute to drug resistance of tumour cells. Many tumour cells of higher stages have also acquired the ability for higher migration and invasion potential. These transformation steps involve changes in intracellular signalling pathways that regulate, amongst other events, actin-based motility. In addition, fast proliferating cells require high supply of energy; therefore deregulation of genes participating in metabolic processes has been described in aggressive tumour cells.

Molecular profiling studies suggested genomic and transcriptional differences in PC cohorts, which may reflect tumourigenesis and disease progression. The prediction of clinically relevant PC by using molecular signatures from patient's tissues or surrogates failed so far, and may be hampered by the heterogeneous intra- and intertumoural genetic background driving the individual disease course. Thus, potentially prognostic markers are often restricted to patient subgroups, for example highly proliferative tumours or cohorts with specific genomic alterations. Nevertheless, such molecular alterations may include oncogenic targets. For example, the stratification of tumours with respect to the TMPRSS1-ERG fusion gene status represents a promising approach for targeted therapies in the future. Other methods for sub-classification have been described in recent reports. These indicate that translocations fusing the strong androgen-responsive gene, TMPRSS2, with ERG or other oncogenic ETS factors may facilitate PC development. It has been proposed that the presence or absence of this genetic rearrangement may be used, much like the Gleason grading system, as a diagnostic tool to extract prognostically relevant sub-classifications of this cancer.

It is therefore an object of the present invention to provide means for diagnosing PC in a sample taken from a PC patient (i.e. an individual having or being suspected to have or being at risk to have or acquire PC). These means should preferably be reliable, suitable for routine testing and have high sensitivity and high specificity to enable testing of a large number of samples and eventually be carried out by automatic devices. Preferably, such means should be suitable for distinguishing between different Gleason Patterns, especially between Gleason Pattern 3 and Gleason Pattern 4; and/or between high risk tumours (with high progression risk) and low risk tumours.

Therefore, the present invention provides the use of Maternal Embryonic Leucine Zipper Kinase (MELK) as marker for diagnosis of prostate cancer (PC) in a tissue sample or in a body fluid sample, especially for the diagnosis of high-risk PC.

More specifically, the present invention provides the use of MELK for distinguishing between Gleason Pattern 3 (CA3) regions and Gleason Pattern 4 (CA4) regions in a PC sample.

The present invention is also drawn to the use of MELK for identifying PC stem cell-like tumour cells with high proliferative capacity in a tissue sample or in a body fluid sample. Finding such stem cell-like tumour cells represents another risk parameter for tumour progression and is therefore highly relevant, especially for monitoring tumour patients.

With the present invention, also a method for diagnosing PC, especially high-risk PC, in a patient is provided, comprising the following steps:
- determining the amount of MELK or the amount of expression of MELK in a tissue sample or a body fluid sample of said patient; and
- comparing the amount of MELK or the amount of expression of MELK in the sample with a sample of known PC status.

In the course of the present invention, MELK was identified to be highly correlated with cell cycle associated genes like UBE2C, TOP2A, CCNB2 and AURKB. Marker function of MELK for PC, especially high-risk PC was shown in validation and functional experiments. For MELK gene expression, the association to high-risk PC could be validated in an independent patient cohort (p < 0.005, n = 79). Immunohistochemistry analysis using a tissue microarray (n = 94) revealed increased MELK protein expression in PC tissues of high Gleason score. Knockdown of MELK deceased cell viability in PC3 cells.

MELK (HPK38; KIAA0175; Entrez: 9833; Ensembl: ENSG00000165304; UniProt: Q14680; RefSeq (mRNA): NM_014791; RefSeq (protein) NP_055606) is a human protein encoded on chromosome 9 (36.56 - 36.67 Mb). The gene is transcribed into a 2501 b long m-RNA (NM_014791) and translated into a 651 aa long polypeptide (NP_055606).

Serine/threonine kinases represent a valid protein class for targeted therapies using specific small molecule inhibitor. MELK was described as potential anticancer target in diverse tumour entities (Gray et al., Cancer Res. 2005; 65: 9751-9614). An increase of MELK expression was described in more aggressive forms of astrocytoma (Marie et al., Int J Cancer. 2008; 122: 807-815), breast cancer (Pickard et al., Breast Cancer Res. 2009; 11(4): R60), hepatocellular carcinoma (WO 2009/126271 A1) and melanoma (Ryu et al., PloS One. 2007; 2: e594). MELK knockdown decreased the transformed phenotype of multiple tumour cells lines as measured by both in vitro for proliferation and anchorage-independent growth, and in vivo xenograft assays (Gray et al., 2005). The thiazole antibiotic siomycin A was identified as potent inhibitor of MELK activity. Treatment inhibited tumour growth of glioblastoma in-vivo, and especially target glioblastoma-derived brain tumour stem cells. MELK function was described in different species to play a vital role in preimplantation, cell division and growth of the embryo. During development, MELK is activated in highly proliferative cells, for example to regulate neuronal progenitors cells. In tumourigenesis, MELK may be aberrantly reactivated in cancer stem cells to provide a growth advantage for neoplastic cells and to drive tumour progression. Therefore, although MELK was already identified as a putative oncogenic target in other tumour entities, no data have been available for PC before the present invention. According to a specifically preferred embodiment, the present invention provides a highly advantageous diagnostic tool for a specific subset of PC patients, i.e the patients with a high-risk PC, an "aggressive" PC. With the present invention, this subset of PC (patients) can be diagnosed in a specifically reliable and reproducible manner. High-risk PCs show the highest expression values for MELK.

According to the present invention, MELK was identified as a gene/protein which not only serves as a functional PC marker in principle but, more importantly, as a clear and reliable marker for discriminating between high-risk and low-risk PC. Throughout the present specification the terms "significant high-risk PC" and "high-risk PC" (or "high-risk prostate tumour" "high-risk prostate carcinoma" and the like) are used synonymously. The same holds true for "insignificant low-risk PC" and "low-risk PC".

According to the present invention, the PC patients are divided into two subgroups, low- and high-risk. This is a very important division, because high-risk patients have to be subjected to a different tumour treatment regime than low-risk patients. High-risk patients need aggressive treatment regimens, including prostatecomy; for low-risk patients - for the time being - careful observation of the further development of the tumour is sufficient. According to the prior art, the differentiation between high-and low-risk patients was performed by the Gleason scoring. Gleason score of equal to or below 3+4 are defined as low-risk PC; Gleason score of equal to or above 4+3 are defined as high-risk PC. According to the present invention, the division of patients into high- and low-risk group is significantly improved, because a more reliable marker than Gleason score is provided. This enables also a more reproducible diagnosis than Gleason scoring, since Gleason scoring is often dependent on the skills and experience of the individual person conducting the scoring, on the area of the biopsy sample observed and on the actual location from where the biopsy has been taken.

Since with the present invention a PC with a Gleason score of equal to or below 3+4 can be distinguished from a PC with a Gleason score of equal to or above 4+3, it is clear that also benign prostate tissue can be identified with the present invention (and distinguished from high-risk PC) due to the low MELK expression in benign and low-risk PC patients

Within the method(s) according to the present invention, the amount of MELK or the amount of expression of MELK in the sample is compared with a sample of known PC status. This comparison immediately leads to the knowledge whether the detected amount in the sample is higher or lower (or comparable, i.e. equal to) the sample with known PC status. If the amount in the sample is higher than in the known PC status, the PC of the sample is worse or further progressed (more aggressive) than the known PC status. If the amount in the sample is below the amount in the sample with known PC status, the PC of the sample is less aggressive (less risky) than the PC of known status. If the amounts are in the same range, i.e. equal due to the usual measurement deviations in biological samples, the PC status of the sample is the same as the known PC status. If the known PC status is a low-risk PC, any significantly elevated amount indicates high-risk PC status in the sample.

MELK is a gene/protein which is widely known and investigated; detection and quantification of expressed MELK-m-RNA or MELK-protein is therefore a standard technique well available to a person skilled in the art. Preferably, the expression or amount of MELK in the body fluid sample or in the tissue specimen, especially in the Gleason 3 region of a biopsy sample or a prostatectomy sample identified, is determined by immunohistochemical (IHC) methods, by immunofluorescence (IF) methods, by RNA in-situ hybridisation, by reverse transcriptase polymerase chain reaction (RT-PCR), especially quantitative real time RT-PCR (qRT-PCR), or by a combination of these methods. Such samples could preferably be taken by manual or microscopical microdissection. Other methods for determining the level of (expression of) MELK include MALDI-MS (e.g. WO 2009/004576 A (including surface enhanced laser desorbtion/ionization mass spectrometry (SELDI-MS), especially surface-enhanced affinity capture (SEAC), surface-enhanced need desorption (SEND) or surface-enhanced photo label attachment and release (SEPAR) for the determination of proteins in samples for diagnosing and staging of PC)), antibody testing (e.g. WO 2008/031839 (including immunoprecipitation, Western blotting, Enzyme-linked immuno sorbent assay (ELISA), Enzyme-linked immuno sorbent assay (RIA), dissociation-enhanced lanthanide fluoro immuno assay (DELFIA), scintillation proximity assay (SPA) for diagnosing, staging or monitoring PC samples under investigation of specific marker proteins)), and quantitative nucleic acid testing (e.g. in WO 2006/066965 A, especially PCR, LCR and RT-PCR of samples for PC marker mRNA detection and quantification).

Preferred detection agents for MELK are antibodies being specific for MELK. Such antibodies are well available in the art; commercial anti-MELK-antibodies can be purchased also in high purity levels to establish suitable diagnostic kits. In fact, also detection kits for the three protein markers are commercially available. The antibodies to be used according to the present invention can be monoclonal antibodies as well as polyclonal antibodies, i.e. antibodies contained in a polyclonal antiserum, as well as any antibody fragment wherein the binding specificity for MELK is present. Suitable fragments of antibodies include F(ab')2, Fab and Fv fragments. Suitable derivatives of antibodies include scFvs, chimeric and humanized antibodies. Also diagnostic monoclonal antibodies from mice can be applied according to the present invention. These are easy and cost effective to be produced in cell cultures and can easily be standardised for routine testing.

Usually, the amount of MELK protein or the amount of MELK mRNA in the sample is compared with a reference value for this amount with a known PC status, i.e. a value which is known to be a healthy value (i.e. a value not affected by PC) or which is known to be a diseased status with respect to PC, preferably of a given stage of PC.

Such reference, standard or control samples are all useable in principle for comparison with the sample of unknown PC status diagnosed according to the present invention. Such reference, standard or control samples can be taken e.g. from a human male subject with negative diagnosis PC or undetectable ongoing PC development. If such a control sample, standard sample or reference sample is said to be comparable to the sample that is taken from a human male subject being suspected to be afflicted by ongoing cancer development according to the present invention, this means that both samples, except for their expression profile have been derived and treated equally. Thus, the sample in both cases may e.g. be a tissue or blood derived sample which has been further treated in a way to allow for detection of the diagnostic marker molecules as mentioned above.

Although prostate tissue samples from the patient are preferred samples, the present marker is also useable in other suitable samples of tissue or body fluids For example, the sample may be a blood sample, or a sample derived from blood, such as e.g. a serum sample or a plasma sample or a fraction of a blood, serum or plasma sample. Samples, which do not need prostate biopsies or prostatectomy performed on the patient for providing the samples are specifically preferred for early staging and diagnosis of PC.

Blood plasma is the yellow liquid component of blood, in which the blood cells in whole blood would normally be suspended. It makes up about 55% of the total blood volume. It is mostly water (90% by volume) and contains dissolved proteins, glucose, clotting factors, mineral ions, hormones and carbon dioxide. Blood plasma is prepared by spinning a tube of fresh blood in a centrifuge until the blood cells fall to the bottom of the tube. The blood plasma is then poured or drawn off. Blood plasma has a density of approximately 1.025 kg/I. Blood serum is blood plasma without fibrinogen or the other clotting factors (i.e., whole blood minus both the cells and the clotting factors).

The diagnostic methods according to the present invention may also be carried out in saliva, bladder washing, semen or urine samples, especially urine samples after prostate massage.

Although the diagnostic fine tuning for established clinical practice will work with nomograms for defining the differences in marker expression according to the present invention, it is also preferred to establish the diagnostic system with the comparison of the sample to be analysed according to the present invention and a sample of the same source of a known status. It is therefore preferred to use the amounts present of MELK (or the amount of respective MELK mRNA present) in a healthy (prostate) tissue sample and compare these to the (prostate) tissue sample under investigation. If the amount of MELK (or the mRNA) is significantly increased, diagnosis of PC is indicated, especially if taken from a Gleason Pattern 3. The same is preferred for e.g. blood, plasma, serum, urine, semen or saliva samples: also here suitable comparison values are derived from samples blood, plasma, serum, urine, semen or saliva, respectively, from a patient with has a healthy prostate tumour status, preferable samples taken (earlier) from the same patient. On the other hand, the comparison may also be taken to establish known amounts of MELK (or the mRNA) in the sample taken. As usual in human medical diagnosis, absolute limiting value can be defined for each of the samples to determine difference between healthy and prostate tumour and between different stages of the tumour. These absolute values have to be defined and carefully confirmed depending on the very method applied for determining MELK. For the examples according to the present invention, a reliable test system has been established, i.a. based on a scoring system already established for other tumour markers (e.g. the "Remmele score" or quickscore (Detre et al., J. Clin. Pathol., 48 (1995), 876-878) for mammary carcinoma).

The level of expression or the amount of protein present of MELK according to the present invention is measured and is compared with the level of expression of MELK from other cells or samples. The comparison may be effected in an actual experiment; or intellectually (by comparison with known reference Values); or virtually (e.g. automatically in silico). When the expression level (also referred to as expression pattern or expression signature (expression profile)) is measurably different, there is according to the invention a meaningful (i.e. statistically significant) difference in the level of expression. Preferably the difference in protein amount or in expressed mRNA for MELK is at least 5 %, 10% or 20%, more preferred at least 50% or may even be as high as 75% or 100%. More preferred this difference in the level of expression or protein amount is at least 200%, i.e. two fold, at least 500 0, i.e. five-fold, or at least 1000%, i.e. 10 fold. The expression level for each of the markers according to the present invention expressed higher in a disease sample than in a healthy, normal sample is at least 5%, 10% or 20%, more preferred at least 50% or may even be 75% or 100%, i.e. 2-fold higher, preferably at least 10-fold, more preferably at least 50-fold, and most preferably at least 100-fold higher in the disease sample. Whether a MELK level is increased in a given detection method can preferably be established by analysis of a multitude of GS6 tumours with the given detection method. This can then form a suitable level from which the "increased" status can be determined; e.g. by the above % difference or -fold change.

An increased expression of MELK is indicative for a highly aggressive tumour (Gleason pattern 4 or 5 portion) resulting in a higher GS classification. In biopsy, a randomly hit tumour area is contained. Since the PC is often multifocal and heterogeneous, the biopsy area can therefore represent the tumour as a whole, but not necessarily. Diverse studies show that Gleason patterns (especially with respect to Gleason 3 patterns) in a biopsy only identify to an extent to 50 to 60 % of the cases a GS6 tumour, the other cases containing higher Gleason Patterns and being > GS6 tumours.

Preferred embodiments of the present invention rely on the determination of MELK expression levels in different tissue regions with different Gleason pattern of tumour biopsies of PCs. Although the Gleason typing and the Gleason score is well available for the person skilled in the art, a short summary thereof is included herein:
A Gleason score is given to PC based upon its microscopic appearance. Cancers with a higher Gleason score are more aggressive and have a worse prognosis.

Most often, a urologist will remove a cylindrical sample (biopsy) of prostate tissue through the rectum, using hollow needles, and prepare microscope slides. After a prostate is removed in surgery, a pathologist will slice the prostate for a final examination.

The pathologist assigns Gleason grades (also known as Gleason Patterns) to the tumour. The two predominating grades are added together to get a Gleason score (also known as the Gleason sum). For example, if the predominant Gleason Pattern was grade 3, and the next most prevalent pattern was grade 4, the Gleason score would be 3 + 4 = 7. If there is only one dominating Gleason Pattern then the Gleason score is two times this Gleason Pattern, e.g. Gleason Score = 2 x 3 = 6 when Gleason Pattern 3 is the single predominating Gleason Pattern of a tumour.

The Gleason grade ranges from 1 to 5, with 5 having the worst prognosis. The Gleason score ranges from 2 to 10, with 10 having the worst prognosis.

Gleason scores are associated with the following features:
Grade 1 - The cancerous prostate closely resembles normal prostate tissue. The glands are small, well-formed, and closely packed
Grade 2 - The tissue still has well-formed glands, but they are larger and have more tissue between them.
Grade 3 - The tissue still has recognizable glands, but the cells are darker. At high magnification, some of these cells have left the glands and are beginning to invade the surrounding tissue.
Grade 4 - The tissue has few recognizable glands. Many cells are invading the surrounding tissue
Grade 5 - The tissue does not have recognizable glands. There are often just sheets of cells throughout the surrounding tissue.

A pathologist examines the biopsy specimen and attempts to give a score to the two patterns. First called the primary grade, represents the majority of tumour (has to be greater than 50% of the total pattern seen). Second - a secondary grade - relates to the minority of the tumour (has to be less than 50%, but at least 5%, of the pattern of the total cancer observed). These scores are then added to obtain the final Gleason score. For the present invention, tissue samples of prostate patients are therefore the preferred tissue sample wherein MELK expression levels are determined.

Accordingly, the present invention relates to the use of expression levels of MELK in a Gleason 3 region of a tissue sample of a PC for distinguishing significant high-risk PC from insignificant low-risk PC wherein increased expression levels of MELK identify a significant high-risk PC and wherein non-increased or decreased expression levels of MELK identify an insignificant low-risk PC.

The present invention also relates to a method for distinguishing significant high-risk PC from insignificant low-risk PC in a patient comprising the following steps:
- determining the expression of MELK in a prostate tissue sample or in a body fluid sample of said patient; and
- identifying the sample as being derived from a patient with a significant high-risk PC if the expression levels of MELK determined are increased compared to the expression levels of MELK in a sample of an insignificant low-risk PC.

The core of the present invention is to use MELK as a marker in PC diagnostics. This invention can further be combined with already existing and established markers for PC diagnostics, such as PSA (and/or PSA subtypes), PSM (prostate specific membrane antigen), hK2 (human glandular kallikrein 2), AMACR (alpha-methylacyl-CoA racemase), hepsin or PCA3 (PC antigen 3), among many others.

Another aspect of the present invention is to provide a MELK detection and/or quantification kit for use in PC diagnosis, especially high-risk PC diagnosis. Accordingly, the present invention provides the use of a kit for detecting the amount of MELK in a sample, comprising detection agents for MELK for diagnosing a tissue sample or a sample of a body fluid for PC, especially for distinguishing significant high-risk PC from insignificant low-risk PC. The present invention also relates to the use of a kit for detecting the expression level of MELK in a sample, comprising detection agents for MELK mRNA for diagnosing a tissue sample or a sample of a body fluid for PC, especially for distinguishing significant high-risk PC from insignificant low-risk PC.

In general, the kit according to the present invention includes binding agents that specifically bind to MELK protein. These binding agents are preferably linkable or already operably linked to a detectable label. An increased binding of the binding agent specifically bound to MELK protein in the sample of the patient compared to the binding agent bound to MELK protein in a sample of known PC status, e.g. a sample from a healthy individual indicates the PC status of the patient. In this embodiment of the invention, a medical imaging device or system detects the binding agent specifically bound to MELK. Preferably, the binding agent is immobilised on a solid surface in order to allow simple confirmation of the binding event. Examples for such solid surfaces include microtiter plates, microarrays, ELISA plates, etc.. Exemplary binding agents include natural ligands, synthetic small molecules, chemicals, nucleic acids, peptides, proteins, antibodies and fragments thereof. As already stated above, antibodies against MELK and fragments thereof specifically binding to MELK are preferred binding agents according to the present invention.

Exemplary detectable labels include fluorophores, chemical dyes, radioactive compounds, chemiluminescent compounds, magnetic compounds, paramagnetic compounds, enzymes that yield a coloured product, enzymes that yield a chemiluminescent product and enzymes that yield a magnetic product. These labels may be linked to a secondary binding agent, e.g. an antibody specifically recognising the MELK binding agent. Various methods for detection of MELK are disclosed e.g. in US 6,670,166 B1, US 7,413,851 B2 and US 7,276,588 B2 or easily adaptable to MELK.

According to a preferred embodiment, the MELK binding agents are detectable by a different label than other markers which are tested, preferably detectable in one and the same assay, e.g. by different fluorophores, different dyes, different colour developing agent systems, etc..

According to a preferred embodiment for determining the amount of MELK in a prostate tissue biopsy sample, MELK and each additional marker can be detected in consecutive tissue slices (preferably about 4-40 µm thickness). Alternatively, a multiple staining can be performed via immunofluorescence.

For IHC staining, a double staining with P63, a marker for basal cells (Weinstein et al., Mod. Pathol. 15 (2002): 1302-1308) is preferred which allows a discrimination between tumour cells and benign areas.

According to an alternative embodiment of the present invention, a kit for detecting the expression level of MELK in a sample, is provided, comprising detection agents for MELK mRNA.

The kit can comprise suitable primer pairs specific for MELK mRNA and/or reagents enabling a quantitative detection of MELK mRNA in a given sample. The kit may also contain instructions for determining PC diagnosis and prognosis based on the detection of the particular marker combination according to the present invention. The present kits are specifically useful for diagnosing a tissue sample or a sample of a body fluid for PC, especially for distinguishing significant high-grade PC from insignificant low-grade PC.

The kit may alternatively or in combination also comprise specific oligonucleotides hybridising to MELK mRNA. Such specific oligonucleotides may act as probes for the MELK mRNA in the sample and can be used to quantitate the specific mRNA in the sample. Preferably, the oligonucleotide probes can be immobilised, e.g. on a suitable microarray. Also established microarray systems, as e.g. the Affymetrix chip, can be used for the diagnosis according to the present system.

Amplification assays, such as PCR, especially qRT-PCR, may be adapted for real time detection of multiple amplification products (i.e., multiplex real time amplification assays). Suitable primer pairs can be made based on the known sequences of the MELK mRNA available e.g. from the sources mentioned above (NCBI, HGNC, etc.).

Such a kit may further include additional components such as additional oligonucleotides or primers and/or one or more of the following: buffers, reagents to be used in the assay (e.g., wash reagents, polymerases or internal control nucleic acid or cells or else) and reagents capable of detecting the presence of bound nucleic acid probe or primers. Examples of detection reagents include, but are not limited to radiolabelled probes, enzymatic labelled probes (horse radish peroxidase, alkaline phosphatase), and affinity labelled probes (biotin, avidin, or steptavidin). Of course the separation or assembly of reagents in same or different container means is dictated by the types of extraction, amplification or hybridization methods, and detection methods used as well as other parameters including stability, need for preservation etc. It will be understood that different permutations of containers and reagents of the above and foregoing are also covered by the present invention. The kit may also include instructions regarding each particular possible diagnosis, prognosis, theranosis or use, by correlating a combined MELK mRNA level over the MELK mRNA level with a particular diagnosis, prognosis, theranosis or use, as well as information on the experimental protocol to be used.

The methods generally disclosed for detection of PCA3 and PSA mRNA in the WO 2006/066965 A are applicable also for the detection of MELK mRNA levels in the samples according to the present invention, especially for detection these levels in prostate tissue biopsy and blood derived samples.

It is evident that any method or use according to the present invention refers to in vitro methods and uses. Samples are provided by usual diagnostic routes and not entailing a substantial health risk.

Since MELK was also functionally verified as a central target of tumour progression in the present invention, the present invention also relates to use MELK as a therapeutical target. Although marker function of a protein and its use as a target are usually not correlating, the results obtained in the course of the present invention showed that MELK can be directly addressed in PC treatment. Therefore, the present invention also relates to a MELK-inhibitor for use in the therapy of PC, especially high-risk PC. According to the present invention, an effective amount of a MELK inhibitor (or an inhibitor of MELK expression which is referred to herein also as a "MELK inhibitor") is administered to a PC patient, especially a high-risk PC patient. "An effective amount" means that the cumulative amount of the MELK inhibitor stops or at least slows down PC progression and ameliorates the state of the patient. This treatment according to the present invention is most suitable for high-risk PCs, preferably for the therapy of tumours with a high progression risk, e.g. tumours which go into progress after a first-line therapy (e.g. radical prostatectomy) and eventually second-line therapy (e.g. hormone depletion).

Preferred MELK-inhibitors may be selected from an anti-MELK-antibody, a MELK antisense molecule such as a MELK si-RNA (especially an si-RNA pool), a MELK antisense oligonucleotide or similar molecules or mixtures thereof. Administration can be performed in any suitable way for these compounds, e.g. local (intratumoural) as well as systemic (e.g. intravenously) depending on tumour stage and tumour localisation. Although Siomycin A and thiostrepton have been used to inhibit growth and induced cell death in PC cells, these two substances were used as FoxM1 inhibitors (Pandit et al., The Prostate 2011;70:825-833). Those in-vitro results for isolated PC cells therefore did not represent a reasonable expectation of success to combat PC in a patient. Moreover, the specific usability of MELK-inhibitors for high-risk PCs was only revealed with the present invention. Therefore, it is preferred to use anti-MELK-antibody, a MELK antisense RNA, a MELK si-RNA, Siomycin A, thiostrepton, or mixtures thereof for the treatment of high-risk PC patients.

The invention is further described by the following examples and the figures, yet without being limited thereto:
Figure 1 shows hierarchical clustering of 144 Gleason-associated genes from microarray analysis upon LIMMA test indicates a good separation of the two risk subgroups.
Figure 2 shows similar expression pattern of MELK (A, C) and proliferation marker Ki 67 (B, D) protein in high-grade PC. High MELK expression in single tumour cells supports association to proliferation status or stem cell incidence.
Figure 3 shows qRT-PCR analysis of MELK transcript in 79 PC and corresponding benign tissues. MELK transcript is significantly more abundant in tumour versus benign tissues (A), in high-grade vs low-grade tumours with respect to Gleason score (B) or pT stage (C), but not associated to PSA density (D) (y-axis: Δ Cₚ value normalised using B2M).
Figure 4 shows differential gene expression of MELK could be verified in an independent dataset reported by Taylor et al. MELK expression was not only upregulated in tumour versus prostate benign tissues (A) (y-axis: normalised microarray expression value (log expression value), but also associated to a bad outcome in PC (B).
Figure 5 shows expression analysis of MELK protein by IHC revealed high expression in high-grade PC (A) intermediate expression expression in low-grade PC (B) and PIN precursor lesions (C), and low expression in benign prostate epithelium with (D) MELK expression scoring indicates increased expression in high grade PC, lower levels in benign and low-grade cases and intermediate expression in premalignancies (PIN) (E, F).
Figure 6 shows correlation between UBE2C and MELK transcripts across 98 PC and benign tissues (y-axis: normalized microarray expression value of Illumine data (log expression value)).
Figure 7 shows RNA interference based knockdown of UBE2C and MELK expression in PC3 and LNCaP cells. UBE2C and MELK expression was decreased to about 30% transcriptional activity (A, C). For both genes, cell viability was decreased upon knockdown across different time points (B, D).
Figure 8 shows a microarray experiment after MELK knockdown conducted in triplicates revealed 345 significantly expressed genes (A). Decreased genes indicate MELK downstream biological processes like chromatin modification, embryogenesis and cell migration.

### Examples:

In the present examples, a screen for genes stratifying high-risk and low-risk PC was performed. Several identified genes were associated to cell cycle progression, and MELK was further validated to verify the relevance as diagnostic and therapeutic target in PC according to the present invention.

### Materials and Methods:

### Tissue Specimens

Frozen (n=98) and paraffin-embedded (n=158) prostate tumour and benign tissue samples were obtained from previously untreated patients, who had undergone radical prostatectomy after tumour diagnosis in a PSA based screening program performed in Tyrol by the Department of Urology, Medical University of Innsbruck (Oberaigner et al., Int J Public Health. 2011; in press). The study was approved by the ethics committee at the Medical University of Innsbruck. Immediately after surgery, the prostate specimens were rapidly processed for histopathological analysis and storage of snap-frozen and fixed, paraffin-embedded tissue (Bu et et al., Prostate. 2011;71:575-87). Tissue samples were isolated from a cohort with Gleason scores (GS) between 6 and 10 (Table 1).

**Table 1. Clinical and Demographic Characteristics of the Patient Cohorts**

| Parameter | | Screening cohort | | Valiation cohort | |
|---|---|---|---|---|---|
| | | Tumour (n=59) | Benign (n=39) | Tumour (n=79) | Benign (n=79) |
| Gleason score | | | | | |
| | 5, 6 | 5 | NA | 40 | NA |
| | 7 (3+4) | 28 | NA | 17 | NA |
| | 7 (4+3) | 12 | NA | 4 | NA |
| | 8-10 | 15 | NA | 18 | NA |
| Median age, yr | | 62 ±7.2 | 61 ±7.0 | 60 ±6.2 | 60 ±6.2 |
| Epstein criteria | | | | | |
| | significant | 49 | NA | NA | NA |
| | insignificant | 9 | NA | NA | NA |
| | unknown | 1 | NA | NA | NA |
| Fusion gene status | | | | | |
| | Yes | 31 | NA | NA | NA |
| | No | 26 | NA | NA | NA |
| | unknown | 2 | NA | NA | NA |
| PSA, Biopsy | | 6.0 ±5.1 | NA | 5.4 ±5.5 | NA |

Two subgroups were defined as low- (GS ≤ 3+4) and high-risk (GS ≥ 4+3). Apart from tumour foci, the corresponding benign tissue was collected from a subset of the same specimens. All tumour and benign areas were assigned by an experienced uropathologist, and tissue samples were isolated by macrodissection of 10µm cryo- or FFPE sections.

### Microarray analysis and Data mining

Total RNA from frozen prostate tissues was isolated using EZ1 RNA Mini Kit (Qiagen) according to the manufacture's instruction. The quality of total RNA was checked using the Agilent 2100 Bioanalyzer (Agilent Technologies GmbH, Berlin, Germany). RNA concentrations were determined using the NanoDrop spectrophotometer (NanoDrop Technologies, Wilmington, DE).

Samples were prepared for hybridization on Illumina Human Sentrix-12 BeadChip arrays (Illumina, Inc.) according to Illumina's recommendations. In brief, 250 ng total RNA was used for complementary DNA (cDNA) synthesis, followed by the synthesis of biotin-labeled cRNA according to the MessageAmp II aRNA Amplification kit (Ambion, Inc., Austin, TX). Hybridization, washing, drying and scanning of the microarrays was performed according to the manufacture's instruction.

Data analysis was done by normalization of the signals using the quantile normalization algorithm without background subtraction. Differentially expressed genes between sample subgroups were selected by Significance Analysis of Microarrays (prostate tissue dataset) and LIMMA (cell line experiments), followed by the analysis for under- and overrepresentation of genes in distinct gene ontologies by using GOstat as described in a previous study (Pressinotti et al., Mol Cancer. 2009;8:130). Hierarchical clustering was done using Euclidian distance measurement. Coexpression of genes was analysed using Spearman correlation. Kaplan-Meier survival curve was calculated by separating the patient cohort of a reported microarray dataset (Taylor et al., Cancer Cell. 2010;18:11-22) according to the median MELK expression. The reported event and time to biochemical relapse (PSA) was taken as the endpoint in this analysis.

### Quantitative real-time RT-PCR validation

Independent qRT-PCR validation was performed using FFPE prostate tissue samples. Total RNA from FFPE tissues was isolated by RNeasy FFPE Kit (Qiagen) with initial modifications. Deparaffination and lysis procedure was done via heating (60°C, overnight). All quantitative measurements of specific genes were performed on the LightCycler 480 (Roche) using Taqman primer and probe assays (Applied Biosystems). Cp values of target genes were normalized using a housekeeping gene (B2M). Furthermore, a second housekeeping gene (POLR2A) was analyzed together with the candidate genes. Relative quantification of target genes was calculated by the 2^{-Δ Δ Ct} method. Gene expression variance between different groups was analyzed by Wilcoxon test. A list of examined genes including mean Ct values of each analyzed group and corresponding sequences is given in Table 2.

**Table 2: Downstream processes assessed to the downregulated genes upon MELK and UBE2C RNAi-based knockdown or overlapping genes between both experiments..**

| | | | | | |
|---|---|---|---|---|---|
| MELK Genes downregulated | | | | | |

| GO ID | GO Name | Genes | Group count | Total count | p-value |
|---|---|---|---|---|---|
| GO:0016568 | chromatin modification | carm1; crebbp; setd2; trrap; myst4; arid1a; chd8; smarcc2; smarca4 | 9 | 219 | 2.10E-05 |
| GO:0009790 | embryonic development | lama5; slit2; foxc1; fbn2; vegfc; Irp5; lama3; chd8; smarca4 | 9 | 235 | 3,42E-05 |
| GO:0016573 | histone acetylation; | myst4; trrap; crebbp | 3 | 13 | 0,000454 |
| GO:0016477 | cell migration | lama5; Irp5; vegfc; lama3; nr2f1; slit2; foxc1 | 7 | 233 | 0,00104 |
| UBE2C Genes downregulated | | | | | |

| GO ID | GO Name | Genes | Group count | Total count | p-value |
|---|---|---|---|---|---|
| GO:0016568 | chromatin modification | carm1; trrap; ep400; smarcc2; fbxl11; smarca4; setd2; setd1a a | 8 | 219 | 0,000969 |
| GO:0030029 | actin filament-based process | abl1; myo5a; c14orf173; cdc42bpb; actn4; myo9b; cdc42bpa; myh9; sptan1 | 9 | 260 | 0,000583 |
| GO:0007067 | mitosis | aspm; clip1; shc1; cdc2l1; cenpe; numa1; ube2c; zzef1 | 8 | 239 | 0,00149 |
| GO:0007155 | cell adhesion | clstn1; itga3; lama5; abl1; col6a2; inppl1; zyx; itgb4; ptprm; sirpa; lpp; ptprf; col6a1; cd99 | 14 | 960 | 0,00317 |
| GO:0022403 | cell cycle phase | aspm; abl1; clip1; shc1; cdc211; cenpe; numa1; ube2c; zzef1 | 9 | 369 | 0,00317 |
| MELK and UBE2C Genes downregulated | | | | | |

| GO ID | GO Name | Genes | Group count | Total count | p-value |
|---|---|---|---|---|---|
| GO:0016568 | chromatin modification | carm1; trrap; smarcc2; setd2; smarca4 | 5 | 219 | 0,00788 |
| GO:0009790 | embryonic development | Irp5; lama5; slit2; smarca4; fbn2 | 5 | 235 | 0,00837 |

### Immunohistochemical Analysis

For validation of expression at the protein level, immunohistochemistry (IHC) on corresponding paraffin-embedded tissue specimens from the same patient cohort was used. For statistical calculation of expression a tissue microarray (TMA) containing standard 0.6 mm tissue cores of 94 radical prostatectomy cases (3 tumour and 1 benign cores for each case) was used. For expression analysis of MELK protein the polyclonal rabbit antibody HPA017214 (Sigma) was used at a dilution of 1.2 µg/mL. Immunohistochemistry was performed with 5 µm paraffin tissue sections employing the Ventana Discovery - XT staining automat (Roche). Standard deparaffination and antigen retrieval by autoclaving in citrate buffer pH 6 for 30 min at 120°C was followed by incubation with antibody solution for 1 hr, universal antibody ready to use secondary antibody solution for 32 min, staining with DAP map kit and counter stain for 4 min with haematoxylin II bluing reagent (all from Roche). Specificity of staining was controlled by including a control antibody (DAKO Cytomation, Glostrup, Denmark) and cores of different cultured cell lines. Immunoreactivity was then scored by a uropathologist and stratified according to the histology and the Gleason pattern of the specimens using a 4 point scaling system: 0, no staining, 1, weak staining, 2, intermediate staining, 3, strong staining (Bu et al., 2011).

### RNA interference and cell viability assay

The human PC cell line PC3 was cultured in RPMI 1640. The medium was supplemented with 50 units/ml penicillin, 50 µg/ml streptomycin sulphate, 1% nonessential amino acids, and 10% FBS (all from GIBCO/BRL, Gaithersburg, MD, USA). The assay is also performed in LNCaP cells. For knockdown experiments siRNA pools for *MELK, UBE2C* and a scrambled siRNA control (Table 2) was purchased from Dharmacon (Lafayette, CO, USA). The siRNA knockdown experiments were performed by plating 0.8 x 10⁴ (0.5 x 10⁵) PC3 cells in a 96-well plate (6-well plate) overnight. For transfection, siRNA (50nM) and HiPerfect (Qiagen) were diluted separately and incubated for 5 min at room temperature. The two solutions were mixed and incubated for 20 min at room temperature. siRNA-HiPerfect mixture was then added to the cells, and the plate was mixed by gentle rocking. Transfected cells were incubated at 37°C and 5% CO₂ for 48 and 72 h. Gene knockdown efficiency was verified on the transcript level by qRT-PCR. Metabolic cell viability assay WST1 (Roche) was performed in four replicates upon target knockdown. For microarray experiments, the gene knockdown procedure was performed in 6-well plates (0.5 x 10⁵ cells) in triplicates for 48h.

### Data deposition

The microarray data sets reported herein have been deposited MIAME compliant to NCBI Gene Expression Omnibus (GEO) database (accession no. GSEXXXXX).

### Results:

Gene expression pattern differentiates high- and low-risk PC patients

Microarray data analyses of 59 PC and 39 benign tissue samples revealed major transcriptional differences. More than 5.000 genes have been identified to be differentially expressed between matched tumour and benign samples from 39 patients. The gene signature was comparable to previous studies, and included well known PC target genes (e.g. *AMARC, GDF15, TARP, MYC*). However, the main focus was the identification of candidate genes associated to the disease progression. High-risk tumours of Gleason score (GS) ≥ 4+3 (n = 27) were compared with low-risk tumours of GS ≤ 3+4 (n = 32). In total, 144 differentially expressed genes were identified (FDR < 0.05), 80 upregulated and 64 downregulated genes in high-risk tumours.

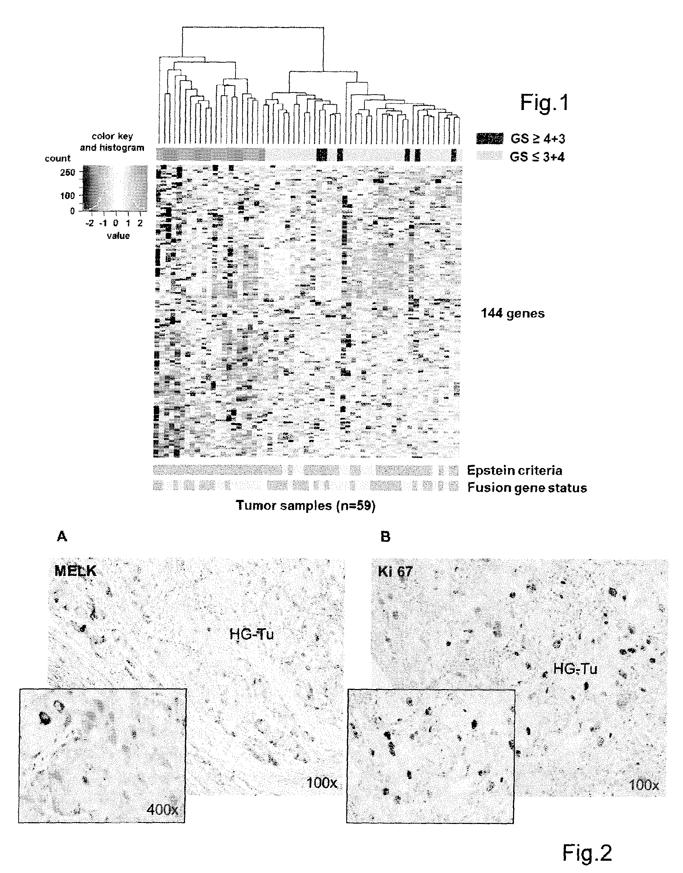

The stratification of high- and low-risk tumours could be fairly reflected by hierarchical clustering (Figure 1). The majority of high-risk tumours with GS ≥ 4+3 (21/27) was characterized by transcriptional similarities within one cluster. In total 14 out of 15 tumours with Gleason 8 or higher were included in the cluster of high-risk tumours. But, only half of the Gleason 7 tumours with a dominant Gleason pattern 4 (GS 4+3) reflected transcriptional pattern of high-risk tumours. The second main cluster included all clinically assessed low-risk tumours GS ≤ 3+4 (32/32). Of note, molecular profiling of Gleason 7 tumours with a dominant Gleason pattern 3 (GS 3+4) more homogeneously reflected low-risk pattern.

Comparing the clinically assessed Gleason score against the Gleason pattern, which was assessed in the tumour foci used for microarray analysis discrepancies were detected in 7 out of 59 cases. In 6 out of 7 cases the Gleason score in the tumour foci was lower than the clinically assessed one. In three cases, the different GS annotation would have consequences on the present risk-group allocation. Here, the indicated low-risk Gleason pattern based on the specifically used tumour foci was better reflected in the hierarchical clustering. Other tumour characteristics like fusion gene status of TMPRSS2/ERG or Epstein criteria did not reveal a strong impact on the clustering (Figure 1). For example, diverse stratification of Gleason 7 tumours with a dominant Gleason pattern 4 in both clusters could not be explained by those parameters.

Gene ontology analysis revealed distinct biological processes for which an overrepresentation of genes associated to GS was stated (Table 3).

**Table 3: Gene ontology statistics of the upregulated genes in tumors with higher Gleason score indicated distinct gene ontologies like cell cycle.**

| GO ID | GO Name | Genes | Group count | Total count | P-value |
|---|---|---|---|---|---|
| GO:0007049 | cell cycle | cdca8; prc1; pttg1; cdc20; aurka; cenpe; stmn1;jag2; cdc451; aurkb; ube2c; e2f2; gspt1; nusap1; uhrf1 | 15 | 839 | 3.78E-08 |
| GO:0051301 | cell division | cdca8; prc1; pttg1; cdc20; cenpe; aurkb; ube2c; nusap1 | 8 | 245 | 3.41 E-06 |
| GO:0007051 | spindle organization and biogenesis | ube2c; prc1; aurka; stmn1 | 4 | 19 | 3.81 E-06 |
| GO:0030154 | cell differentiation | nrp1; myt1; ngfb; asf1b; cfl1; eef1a2; edaradd; fastk; stmn1; jag2; e2f2; gspt1; rtkn; mapk8ip2; adora1; top2a | 16 | 1810 | 2.61 E-05 |
| GO:0007017 | microtubule-based process | ube2c; prc1; kif20a; aurka; cenpe; nusap1; stmn1 | 7 | 315 | 0.000113 |
| GO:0006915 | apoptosis | rtkn; cfl1; eef1a2; mapk8ip2; adora1; fastk; top2a; e2f2; gspt1 | 9 | 855 | 0.001250 |
| GO:0019953 | sexual reproduction | brd2; jag2; spag1; asf1b; pttg1 | 5 | 315 | 0.006920 |

For example, when analyzing all 80 significantly upregulated genes in tumours of higher GS, 15 genes were found to be insolved in cell cycle process. A significantly increased number of genes were also linked to cell differentiation and spermatogenesis. For the validation study, genes upregulated in high-grade PC and strongly associated to cell cycle proliferative function like *CDC20, CDCA8, CCNB2, UBE2C* and *PRC1* were selected. For the 64 downregulated genes, gene ontology statistics was negative.

MELK gene and protein expression is associated with high-risk PC

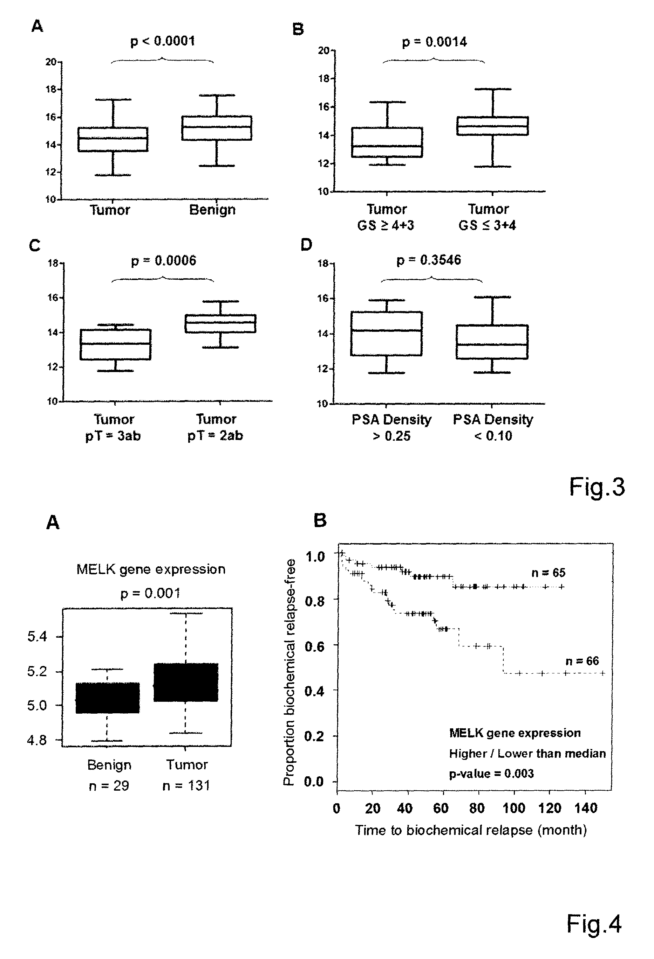

The gene MELK was identified as one of the most significant genes upregulated in high-risk PC. Furthermore, MELK was highly correlated with cell cycle genes like *UBE2C, KIF20A,* TOP2A and *AURKB* (Figure 2). Based on these findings and previous reports, potential MELK function in PC progression and cell cycle proliferation was expected. MELK was therefore included into the validation study. The validation of GS-associated genes was performed by qRT-PCR using FFPE-tissues of an independent patient cohort. Most of the candidate genes could not be verified with respect to the differences in GS. The gene *CDC20* was differentially expressed when the tumour staging was taken into account. All genes were significantly differential expressed between tumour and benign tissues, but not associated to Gleason score or other risk parameters. In contrast, the gene expression variance of *MELK* across diverse PC could be verified in this study (Figure 3). *MELK* expression was increased in tumours compared to benign tissues (p < 0.0001), increased in tumours of higher GS (p = 0.00140) and increased in tumours of higher staging (p = 0.00060). Furthermore, an independent PC microarray gene expression dataset including follow-up data (Taylor et al., 2010) was considered. Here, a higher *MELK* expression was associated with prostate malignancy and early PSA biochemical relapse (p = 0.003, Figure 4A and 4B).

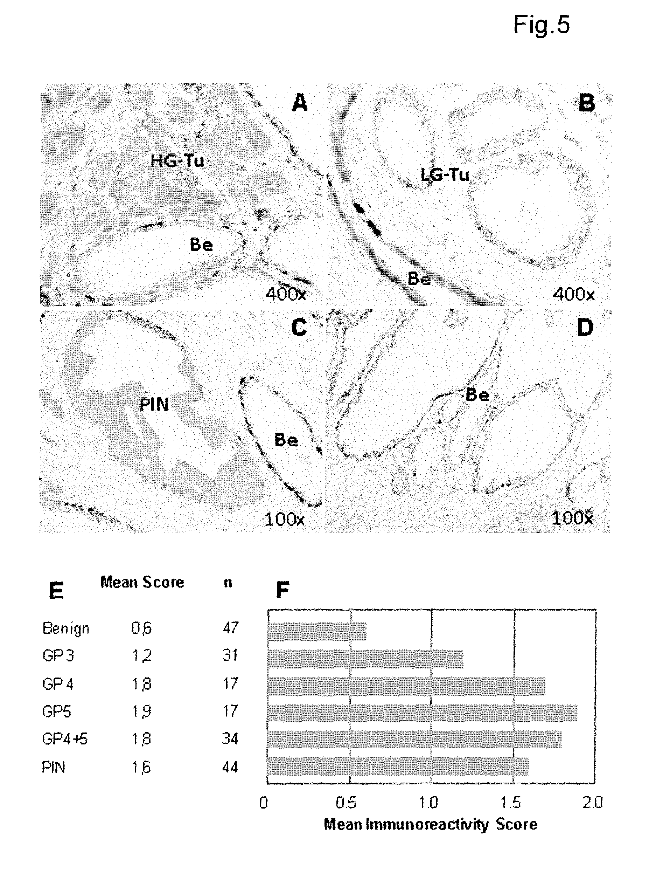

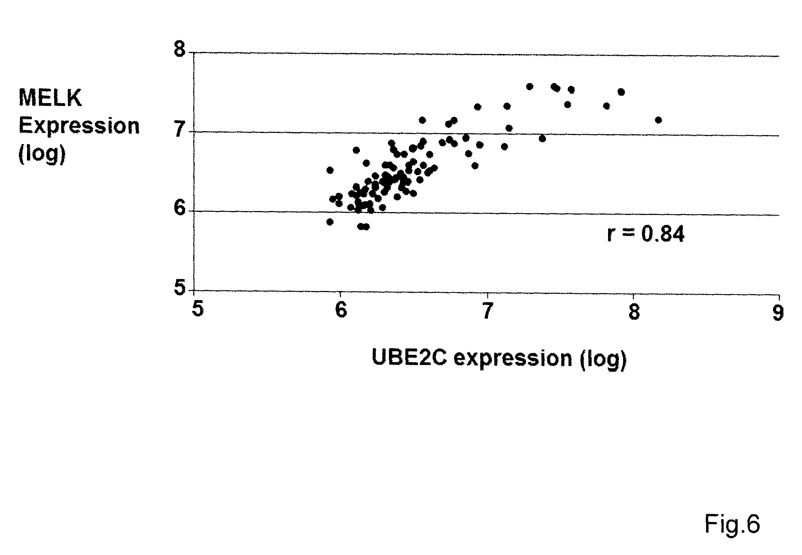

The *MELK* transcript level variance in PC could also be confirmed on the protein level. Immunohistochemistry analyses using a tissue mircroarray of 94 radical prostatectomy cases revealed stronger MELK protein expression in tumours compared to benign samples, and more important a higher expression in tumour samples with higher GS (Figure 5 A-F). Benign and low-grade cases showed low levels, premaligancies intermediate levels of MELK. Highest MELK expression was found in tumour foci with Gleason pattern 4 and 5. Notably, the expression pattern of MELK was not equally distributed across the tumour area. High MELK expression was found in single tumour cells indicating intratumoural heterogeneity (Figure 6). Similar expression pattern of MELK and proliferation marker Ki 67 protein was observed in high-grade PC. This MELK expression pattern indicated association to proliferation status or stem cell incidence.

Downstream affected genes upon *MELK* and *UBE2C* inhibition indicated common involvement in embryogenesis and chromatin modification

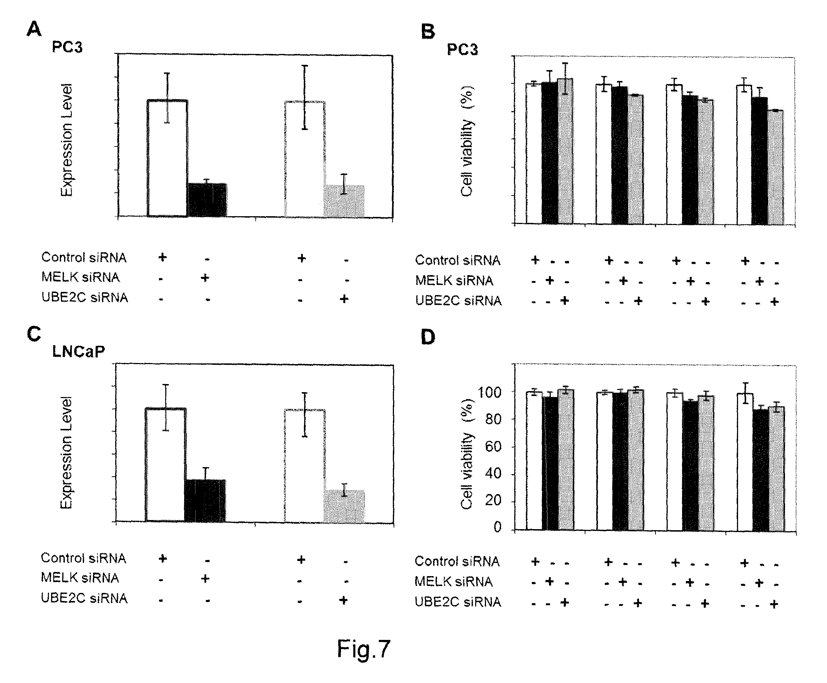

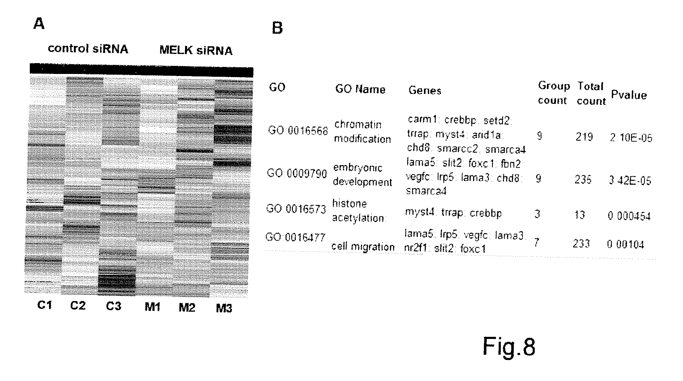

In order to address putative role of *MELK* in PC development and progression, functional analysis upon siRNA-based knockdown was performed, as well as a search for downstream genes and processes by microarray experiments. Because of the coexpression in PC tissues and the functional links of *MELK* and *UBE2C* (cell cycle, proliferation and stem cell marker), both genes were separately inhibited in order to identify potential gene-gene interaction and common oncogenic downstream pathways. RNAi-based inhibition of *MELK* and *UBE2C* was efficient in PC3 PC and LNCaP cells and decreased transcriptional level down to about 30% remaining activity (Figure 7A, C). The cell viability was slightly reduced upon knockdown of MELK or UBE2C after 48h and 72h (Figure 7B, D). Microarray analysis upon knockdown of *MELK* compared to control transfection (non-target control) revealed 345 differentially expressed genes. The heatmap of significantly deregulated genes indicated consistent expression pattern across the experimental triplicates (Figure 8A). The downregulated genes were significantly associated to the gene ontologies chromatin modification, embryogenesis and cell migration (Figure 8B). Furthermore, the knockdown of *UBE2C* affected 361 genes including downregulated targets linked to chromatin modification, cell adhesion and cell cycle. Of note, the target genes *MELK* and *UBE2C* themselves were the most significantly downregulated genes in each experiment. A striking proportion of downregulated genes overlapped upon *MELK* and *UBE2C* knockdown (94 of 299 genes, 31%), and indicated a common regulatory interaction. For example, *MELK* and *UBE2C,* which are reported to play a role in developmental processes and stem cell function, appeared to affect important genes in embryogenesis (*SLIT2, LRP5, SMARCA4)* and in chromatin modification *(CARM1, TRRAP* and *SMARCC2).* Thus, the coexpressed PC target genes *MELK* and *UBE2C* showed a strong overlap in putative downstream targets and gene ontologies upon in-vitro inhibition in PC cells.

### Discussion:

After PC diagnosis the further stratification of high-risk patients, which are likely to get a serious disease course and need therapeutic intervention, is still a challenge. Increased screening effort using serum PSA level and biopsies lead to more PC diagnoses. However, this concept is still questionable with respect to the high rate of patients, who are facing a treatment although they have a silent or only slowly progressing disease. In order to assess the tumour progression the Gleason scoring is defined after biopsy and radical prostatectomy. However, different tumour sites in the prostate often have heterogeneous Gleason pattern, and especially for tumours defined as moderate Gleason score 7 it is difficult to predict a high- or low-risk disease course.

Molecular profiling studies hold great promises to better reflect tumour biology, tumour stage and potential disease outcome. In the course of the present invention, many genes were found to be upregulated in high-risk PC and functionally associated with oncogenic functions like cell cycle progression or cell motility. The present examples showed intraindividual variations in the Gleason pattern while comparing different tumour loci. In most cases, molecular profiling reflected Gleason pattern from the primary source that emphasizes the importance of a careful selection and clinical assessment of biomaterial. In contrast to recent studies (Pressinotti et al., 2009), the present cohort included a large set of tumours with variable Gleason pattern 3 and 4. Molecular profiling of Gleason 7 tumours indicated more variation for the potentially high-risk cases with a dominant Gleason 4 pattern (GS 4+3) compared to the tumours with a dominant Gleason 3 pattern (GS 3+4). On reason could be the changing definitions within the Gleason grading system widen the scope of pattern 4 carcinoma.

Recent studies unravelled more robust gene signatures associated to the disease prognosis. The latter proposed a gene signature in PC predicting lethal disease (Penney et al., J Clin Oncol. 2011;29:2391-6). Here, tumours with Gleason score 7 were stratified for high- and low-risk disease course by applying a gene signature derived from GS < 6 and GS > 8 comparison. Comparing the signatures revealed expected variation with regard to the patient cohort diversity, different microarray platforms and differences in the tested prognostic parameters. However, independent studies indicated dominator genes in PC associated to the disease outcome. For example, the majority of cell cycle genes identified to be upregulated in the present cohort of high-risk PC has been also described in independent microarray studies (Penney et al., 2011).

In the present invention the *MELK* was for the first time identified to be upregulated in high-risk PC. qRT-PCR based validation confirmed the present findings of the microarray screening. Furthermore, *MELK* was strongly correlated to the cell cycle gene cluster, which was independently found to be associated to high-risk PC and disease outcome. Inhibition of *MELK* in the PC cell line PC3 indicated the involvement in embryonic developmental processes and cell migration. Immunohistochemistry *MELK* staining of a PC tissue array confirmed the increased expression in advanced tumours on the protein level. Furthermore, the high abundance of *MELK* only in a subset of tumour cells indicated a link to the proliferative status or stem cell characteristics. *MELK* was alreardy identified as putative oncogenic target in other tumour entities, but not described for PC. Especially in malignant brain tumours, *MELK* was comprehensively analysed and suggested as a promising tumour target. *MELK* has been described to be involved in tumour stem cell function. In a recent study, Siomycin A, an inhibitor of FOXM1, has been shown to target cancer stem cells through a MELK-mediated pathway (Nakano et al., Neuro Oncol. 2011;13:622-34). Siomycin A has been also discussed as potential therapeutic option for PC outside the context of *MELK* (Pandit et al., The Prostate 2011;70:825-833.). Here, FOXM1 was suggested as promising target in PC cells using a therapy combination. The results according to the present invention show that *MELK* is a suitable target especially in high-risk PC.

## Claims

1. Use of Maternal Embryonic Leucine Zipper Kinase (MELK) as marker for diagnosis of prostate cancer (PC) in a tissue sample or in a body fluid sample, especially for the diagnosis of high-risk PC.

2. Use of MELK according to claim 1 for distinguishing between Gleason Pattern 3 (CA3) regions and Gleason Pattern 4 (CA4) regions in a PC sample.

3. Use of MELK for identifying PC stem cell-like tumour cells with high proliferative capacity in a tissue sample or in a body fluid sample.

4. Method for diagnosing PC, especially high-risk PC, in a patient comprising the following steps:
- determining the amount of MELK or the amount of expression of MELK in a tissue sample or a body fluid sample of said patient; and
- comparing the amount of MELK or the amount of expression of MELK in the sample with a sample of known PC status.

5. Method according to claim 4, **characterised in that** the expression of MELK is determined by immunohistochemical (IHC) methods, by immunofluorescence (IF) methods, by RNA in-situ hybridisation, by reverse transcriptase polymerase chain reaction (RT-PCR), especially quantitative real time RT-PCR (qRT-PCR), or by a combination of these methods.

6. Method according to claim 4 or 5, **characterised in that** the sample is a prostate tissue sample, a sample derived from blood, a saliva sample, a bladder washing sample, a semen sample or a urine sample, especially a prostate biopsy or a plasma or serum sample.

7. Method for verifying Gleason Pattern 3 in a prostate tissue sample comprising the following steps:
- identifying a Gleason 3 region in a prostate tissue sample;
- determining the expression of MELK in the Gleason 3 region identified; and
- verifying the identified Gleason Pattern 3 of the tissue sample if the expression levels of MELK determined are increased compared to the expression levels of MELK in a benign region or if the expression levels of MELK determined are decreased compared to the expression levels of MELK in a Gleason Pattern 4 region, a Gleason Pattern 5 region or a prostate intraepithelial neoplasia (PIN).

8. Method for distinguishing significant high-risk PC from insignificant low-risk PC in a patient comprising the following steps:
- determining the expression of MELK in a prostate tissue sample or in a body fluid sample of said patient; and
- identifying the sample as being derived from a patient with a significant high-risk PC if the expression levels of MELK determined are increased compared to the expression levels of MELK in a sample of an insignificant low-risk PC.

9. Use of expression levels of MELK in a Gleason 3 region of a tissue sample of a PC for distinguishing significant high-risk PC from insignificant low-risk PC wherein increased expression levels of MELK identify a significant high-risk PC and wherein non-increased or decreased expression levels of MELK identify an insignificant low-risk PC.

10. Use of a kit for detecting the amount of MELK in a sample, comprising detection agents for MELK for diagnosing a tissue sample or a sample of a body fluid for PC, especially for distinguishing significant high-risk PC from insignificant low-risk PC.

11. Use of a kit for detecting the expression level of MELK in a sample, comprising detection agents for MELK mRNA for diagnosing a tissue sample or a sample of a body fluid for PC, especially for distinguishing significant high-risk PC from insignificant low-risk PC.

12. A MELK-inhibitor for use in the therapy of PC, especially high-risk PC.

13. A MELK-inhibitor according to claim 12, wherein the MELK inhibitor is an anti-MELK-antibody, a MELK antisense RNA, a MELK si-RNA, Siomycin A, thiostrepton, or mixtures thereof.
